# EUROPEAN PATENT APPLICATION

(11) **EP 4 520 355 A1**
(43) Date of publication of application: **12.03.2025**
(21) Application number: 24198820.3
(22) Date of filing: 06.09.2024
(51) Int. Cl.: A61L 15/32, A61L 15/42, A61L 15/48, A61L 26/00

(54) **ARTIFICIAL DRESSING, USE OF ARTIFICIAL DRESSING FOR PROMOTING WOUND HEALING AND METHOD OF MANUFACTURING ARTIFICIAL DRESSING**

(30) Priority: 08.09.2023 US 202363581281 P; 04.09.2024 TW 113133453
(71) Applicant: Anti-Microbial Savior BioteQ Co., Ltd., Kaohsiung City 802701 (TW)
(72) Inventor: TSAI, YI-JU, 802701 Kaohsiung City (TW); YEH, YING-TING, 802701 Kaohsiung City (TW); HONG, YI-LING, 802701 Kaohsiung City (TW); BAI, MENG-YI, 802701 Kaohsiung City (TW)
(74) Representative: Ipside

(57) **Abstract**

An artificial dressing, use of artificial dressing for promoting wound healing and method of manufacturing artificial dressing are disclosed. The artificial dressing comprises a gelatin, a polysorbate 20 and a glutaraldehyde for promoting wound healing. The manufacturing method of the artificial dressing comprises the steps of enabling the composition of the gelatin, the polysorbate 20 and the glutaraldehyde to perform a foaming step, a molding step and a freeing step in sequence. The artificial dressing has obvious pores and is capable of absorbing a liquid weight about 25-35 times of its body weight. After thermal disintegration experiments and adhesion tests, disintegration of the artificial dressing is not observed, and in animal experiments, compared with commercially available dressings, the artificial dressing of the invention is capable of accelerating wound healing when applied dryly; and capable of proliferating functional tissues of the wound when applied wetly.

## Description

### Cross-reference to Related Application

This application claims priority from US provisional Patent Application No. 63/581,281, filed on September 8, 2023; and claims priority from Taiwan Patent Application No. 113133453, filed on September 4, 2024, each of which is hereby incorporated herein by reference in its entireties.

### Technical Field

The invention relates to an artificial dressing and a use of the artificial dressing for promoting wound healing.

### Background of the Invention

Diabetic foot ulcers (DFUs) are one of the more serious complications of diabetes, accounting for 25% of the total number of diabetic patients. Among them, 50% of patients require amputation, and the proportion of death within five years after amputation is as high as 50%. Moisture therapy is used on diabetic foot ulcers to adjust the balance of the extracellular matrix and promote wound healing.

Moisture therapy can provide a moist environment for the wound, perform autolytic debridement of the wound, reduce pain, activate collagen synthesis and promote keratinocytes, and the cells can migrate in the wound bed to accelerate wound healing. Moisture therapy heals 3-5 times faster than traditional dressings (such as gauze, bandages, etc.). The dressings commonly used with moisture therapy include foam, hydrocolloid and hydrogel. Foam dressings used with moisture therapy on the market are mainly collagen dressings, but the cost is relatively high.

### Summary of the Invention

A main component used in an artificial dressing of the invention is gelatin, which has the advantages of biocompatibility, low cost, and an ability to absorb water and form gel. In addition, the artificial dressing of the invention is a sheet gelatin dressing (SIP SIP^{™}). The artificial gelatin dressing has obvious pores and is capable of absorbing a liquid weight about 25-35 times of its body weight. After thermal disintegration experiments and adhesion tests, disintegration of the dressing is not observed. In addition, in results of animal experiments, compared with commercially available dressings, the artificial dressing of the invention is capable of accelerating wound healing when applied dryly; and capable of proliferating functional tissues of the wound when applied wetly.

The invention provides an artificial dressing for promoting wound healing, comprising a gelatin, a polysorbate 20 (tween 20) and a glutaraldehyde.

Preferably, the concentration of gelatin is 5% (g/mL, w/v) to 10% (g/mL, w/v), the concentration of polysorbate 20 is 0.005% (mL/mL, v/v) ~ 0.1% (mUmL, v/v), the concentration of glutaraldehyde is 0.01% (mUmL, v/v) ~ 0.3% (mUmL, v/v) to form the artificial dressing.

Preferably, a thickness of the artificial dressing is 1-10 mm.

Preferably, a pore diameter of the artificial dressing is 60-70 µm.

Preferably, a porosity of the artificial dressing is 60%-67%.

Preferably, the wound includes bruises, contusions, cuts, general acute wounds, first/second-level burns, surgical wounds, bedsores and ulcers.

Preferably, the artificial dressing is in a sheet shape.

Preferably, the artificial dressing becoming gelatinous when the artificial dressing being humidified or absorbing tissue fluid.

Preferably, the artificial dressing has a tolerance to high-energy gamma radiation exposure doses of 15 kGy-25 kGy without affecting texture and function.

A method for manufacturing the artificial dressing as claimed in claim 1, comprising: (1) performing a foaming step by mixing a gelatin, a glutaraldehyde and a polysorbate 20 to form a mixture and stirring to foam; (2) performing a molding step by pouring the mixture onto a mold and spreading it flat after completing the foaming step; and (3) performing a freezing step by placing the mold into a pre-cooled (pre-cooling temperature -20°C~-60°C) plate-type freeze dryer for freezing and freeze-drying to form an artificial dressing.

Preferably, a concentration of the gelatin is 5% (w/v)-10% (w/v).

Preferably, a concentration of the polysorbate 20 is 0.005%(v/v)~0.1%(v/v).

Preferably, a concentration of the glutaraldehyde is 0.01%(v/v)~0.3%(v/v).

A method of using a composition in promotion of wound healing, comprising applying the artificial dressing as claimed in claim 1, which is acted as the composition, to a patient's wound.

Preferably, the composition is used for moisture therapy.

A use of a composition in preparing an artificial dressing for promoting wound healing, comprises providing a composition, which comprises a gelatin, a polysorbate 20 and a glutaraldehyde; and performing a foaming step, a molding step, and a freeing step on the composition.

Preferably, the concentration of gelatin is 5% (g/mL, w/v) to 10% (g/mL, w/v), the concentration of polysorbate 20 is 0.005% (mL/mL, v/v) ~ 0.1% (mUmL, v/v), the concentration of glutaraldehyde is 0.01% (mUmL, v/v) ~ 0.3% (mL/mL, v/v) to form the artificial dressing.

Preferably, the composition is capable of: (1) absorbing tissue fluid of the wound; (2) inhibiting proliferation of granuloma of the wound; (3) reducing inflammation of the wound; (4) promoting angiogenesis in the wound tissues; and (5) reducing adhesiveness of the wound to promote wound healing.

Preferably, the wound includes but is not limited to bruises, contusions, cuts, general acute wounds, first/second-level burns, surgical wounds, bedsores and ulcers.

Accordingly, an artificial dressing, use of artificial dressing for promoting wound healing and method of manufacturing artificial dressing provide the following advantages:
(1). The artificial gelatin dressing has obvious pores and is capable of absorbing a liquid weight about 25-35 times of its body weight.
(2). The artificial dressing of the invention is capable of accelerating wound healing when applied dryly; and capable of proliferating functional tissues of the wound when applied wetly.
(3). A main component used in an artificial dressing of the invention is gelatin, which has the advantages of biocompatibility, low cost, and an ability to absorb water and form gel. The cost of the artificial dressing of the invention is far lower than the traditional moisture therapy and dressings.
(4). The artificial dressing of the invention is capable of: absorbing tissue fluid of the wound; inhibiting proliferation of granuloma of the wound; reducing inflammation of the wound; promoting angiogenesis in the wound tissues; and reducing adhesiveness of the wound to promote wound healing.

Other features and advantages of the invention will be disclosed from the following non-limiting detailed description and embodiments.

### Brief description of the Drawings

FIG. 1 shows a manufacturing process of the artificial dressing of the invention.
FIG. 2 shows cross-sectional views of a gelatin dressing cross-linked with different glutaraldehyde concentrations, FIG. 2(A) shows a glutaraldehyde concentration of 0.30%; FIG. 2(B) shows a glutaraldehyde concentration of 0.20%; FIG. 2(C) shows a glutaraldehyde concentration of 0.10 %; FIG. 2(D) shows a glutaraldehyde concentration of 0.05%; and FIG. 2(E) shows a glutaraldehyde concentration of 0.01%.
FIG. 3 is a schematic diagram of the testing of an artificial dressing of the invention, wherein FIG. 3(A) shows the result of thermally disintegrate testing of the artificial dressing, FIG. 3(B) shows the result of adhesion testing of the artificial dressing.
FIG. 4 is a schematic diagram of animal experiments of an artificial dressing of the invention.
FIG. 5 is a schematic diagram of using imaged software to analyze a wound area of a mouse.
FIG. 6 is a statistical diagram of wound areas of mice in different dressing treatment groups from 0 day to 31 days.
FIG. 7 shows H&E staining analysis of animal experimental skin sections (Group A: NC group without dressing), double arrow is wound bed; single arrow is multinucleated leukocytes (neutrophil); circle is granuloma; spindle-shaped light area in the right figure is fibroblasts, scale bar of the left figure is 1 mm, and scale bar of the right figure is 100 µm.
FIG. 8 shows H&E staining analysis of animal experimental skin sections (Group B: commercially available collagen dressing PC group), double arrow is wound bed; single arrow is multinucleated leukocytes (neutrophil); circle is granuloma; spindle-shaped light area in the right figure is fibroblasts, scale bar of the left figure is 1 mm, and scale bar of the right figure is 100 µm.
FIG. 9 shows H&E staining analysis of animal experimental skin sections (Group C: artificial dressing dry application group), double arrow is wound bed; dashed circle is new functional tissues; solid circle is new blood vessel; light area in the right figure is collagen; spindle shape is fibroblasts, scale bar of the left figure is 1 mm, and scale bar of the right figure is 100 µm.
FIG. 10 shows H&E staining analysis of animal experimental skin sections (Group D: artificial dressing wet application group), double arrow is wound bed; short arrow is monocytes; long arrow is new functional tissues, scale bar of the left figure is 1 mm, and scale bar of the right figure is 100 µm.

### Detailed Description of Embodiments

In order to understand the technical features, content and advantages of the disclosure and its achievable efficacies, the disclosure is described below in detail in conjunction with the figures, and in the form of embodiments, the figures used herein are only for a purpose of schematically supplementing the specification, and may not be true proportions and precise configurations after implementation of the disclosure; and therefore, relationship between the proportions and configurations of the attached figures should not be interpreted to limit the scope of the claims of the disclosure in actual implementation. In addition, in order to facilitate understanding, the same elements in the following embodiments are indicated by the same referenced numbers. And the size and proportions of the components shown in the drawings are for the purpose of explaining the components and their structures only and are not intending to be limiting.

Unless otherwise noted, all terms used in the whole descriptions and claims shall have their common meaning in the related field in the descriptions disclosed herein and in other special descriptions. Some terms used to describe in the present disclosure will be defined below or in other parts of the descriptions as an extra guidance for those skilled in the art to understand the descriptions of the present disclosure.

The terms such as "first", "second", "third", "fourth" used in the descriptions are not indicating an order or sequence, and are not intending to limit the scope of the present disclosure. They are used only for differentiation of components or operations described by the same terms.

Moreover, the terms "comprising", "including", "having", and "with" used in the descriptions are all open terms and have the meaning of "comprising but not limited to".

The invention provides an artificial dressing suitable for promoting wound healing, which comprises a composition of a gelatin, a polysorbate 20 (tween 20) and a glutaraldehyde. The composition is used in preparing an artificial dressing for promoting wound healing. The composition can be applied wetly or dryly. A thickness of the artificial dressing is 1-10 mm. A pore diameter of the artificial dressing is 60-70 µm. A porosity of the artificial dressing is 60%-67%. The artificial dressing is in a sheet shape. The artificial dressing becoming gelatinous when the artificial dressing being humidified or absorbing tissue fluid. The artificial dressing has a tolerance to high-energy gamma radiation exposure doses of 15 kGy-25 kGy without affecting texture and function. The wound includes, but is not limited to, bruises, contusions, cuts, general acute wounds, first/second-level burns, surgical wounds, bedsores and ulcers (such as diabetic foot ulcers).

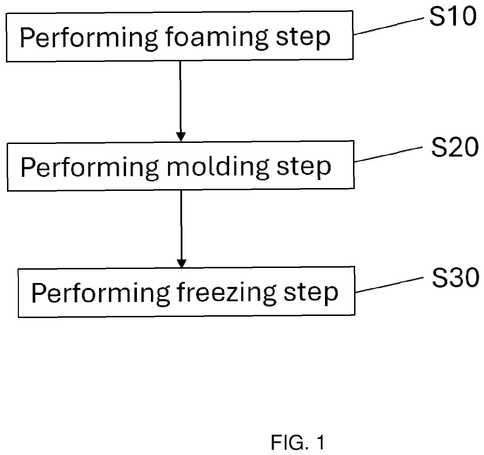

### Embodiment 1: Production process of artificial dressing, as shown in FIG. 1.

A method for making an artificial dressing comprises following steps:
(1) performing a foaming step S10 by mixing 5% (g/mL, w/v) to 10% (g/mL, w/v) gelatin, 0.005% (mUmL, v/v) to 0.1% (mL/mL, v/v) polysorbate 20 (tween 20) and 0.01% (mUmL, v/v) to 0.3% (mUmL, v/v) glutaraldehyde to form a mixture and stirring to foam; The concentration of gelatin, polysorbate-20 and glutaraldehyde mentioned above can be any value and any range among the corresponding values mentioned above, and the rest is water. The gelatin referred to in the process of preparing an artificial dressing of the invention is an aqueous gelatin solution, wherein the concentration of gelatin is 5% (g/mL, w/v) ~ 10% (g/mL, w/v), and may be any value and any range within the range of such values. The invention is for example (but not limited to) using a commercially available mixer (particle emulsifying homogenizer, HMB-312SS, of Chuan Hwa Mi Corporation) to carry out the above mentioned mixing and foaming steps. The stirring time of the above stirring and foaming step is for example (but not limited to) 10 minutes to 15 minutes, and the stirring speed is for example (but not limited to) 1,500 rpm to 3,000 rpm. The stirring time and stirring speed of the present invention can be any value and any range of the above mentioned values, or even other values or ranges other than the above mentioned values, as long as it can make the above mentioned compositions (gelatin, glutaraldehyde and polysorbate-20) evenly mixed and foamed, all of them fall within the scope of the present invention seeking protection. The sources of the above compositions are, for example, commercially available products. For example, gelatin was purchased from Sigma-Aldrich (G2500), glutaraldehyde was purchased from Tokyo Chemical Industry (TCI) (G0068), and polysorbate-20 was purchased from Sigma-Aldrich (P1379), but not limited thereto.
(2) performing a molding step S20 by pouring the mixture onto a mold and spreading it flat after completing foaming, wherein the mold of the invention is not limited to a particular form or style, but may be of any form or style, wherein the mold is a container with a holding space; and
(3) performing a freezing step S30 by placing the mold into a pre-cooled (pre-cooling temperature -20°C~-60°C) freeze dryer for freezing and freeze-drying to form an artificial dressing. The above mentioned freeze dryer is a 15-liter panel-type freeze dryer, Model No. TYFD80015, purchased from Taiyo Yee Industrial Co. After freeze-drying, the composition of the artificial dressing contained 92.6% (g/mL, w/v) to 99.8% (g/mL, w/v) gelatin, 0.05% (mL/mL, v/v) to 1.85% (mUmL, v/v) polysorbate 20 (tween 20), and 0.15% (mUmL, v/v) to 5.55% (mUmL, v/v) of glutaraldehyde. Similarly, the concentration of gelatin, polysorbate-20 and glutaraldehyde in the freeze-dried dressing can be any of the above values and any range.

The freezing process is completed according to operating steps and freeze-drying parameters of the plate-type freeze dryer. The freeze-drying parameters of the freeze dryer are as shown in Table 1.

**Table 1: Freeze-drying parameters of plate-type freeze dryer**

| Group | 1 | 2 | 3 | 4 | 5 | 6 | 7 | 8 | 9 |
|---|---|---|---|---|---|---|---|---|---|
| Temperature | -40°C | -40°C | -20°C | -20°C | 0°C | 0°C | 20°C | 20°C | 30°C |
| Time | 40 mins | 20 mins | 40 mins | 20 mins | 40 mins | 20 mins | 40 mins | 20 mins | 16 hrs |

Although the freeze-drying times for the above mentioned freeze-drying step S30 are listed in Table 1, the invention is not limited thereto, and any freeze-drying time is within the scope of the present invention as long as a freeze-drying artificial dressing can be obtained. For example, the freeze-drying time described above is, for example, greater than 1 hour.

### Embodiment 2: Scanning electron microscopy (SEM) of artificial dressing

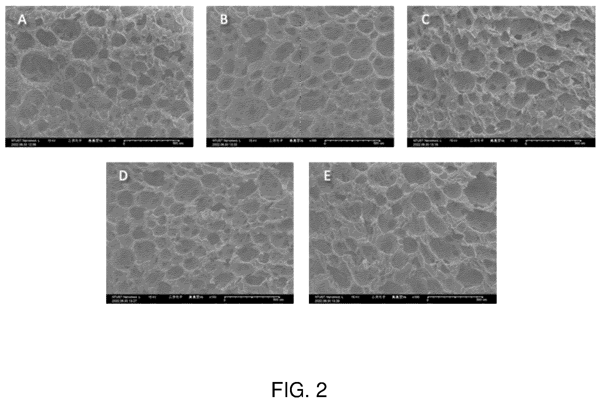

The prepared artificial dressing is used as a sample to be tested, cut the sample to be tested longitudinally (section view), fix the sample to be tested on a special carrier platform with copper tape, and move it to a gold plating machine and vacuumize to 40 Pa and plate with platinum for 60 seconds to increase an electrical conductivity of the sample to be tested. Place the carrier platform containing the sample to be tested into a sample chamber of a scanning electron microscope and adjust a working distance of the carrier platform. After vacuumizing the sample chamber, an image of the sample to be tested can be observed through a screen. Adjust the three-dimensional coordinates XYZ axes knob to align a position of the carrier platform with the field of view, scan the sample to be tested with an electron beam intensity of spot size 50 and a voltage of 15 kV, observe surface shape and structure of the artificial dressing as shown in FIG. 2, and then capture recorded images.

### Embodiment 3: Porosity of artificial dressings

Import the SEM images of embodiment 2 into traditional commercial imaged software to estimate pore diameter and porosity of the artificial dressings with different glutaraldehyde concentrations. Use an image scale to correct and convert a pixel ratio of each image file, adjust a threshold interval of image light and dark contrast, eliminate excessively small noise shadows, and circle pore area size to be calculated, so that average pore diameter and porosity area of the artificial dressing can be estimated and calculated. A porosity of the artificial dressing can be calculated from pictures obtained by SEM by using the software imaged, as shown in Table 2.

**Table 2: Pore diameter analysis results of artificial dressings in SEM images**

| Glutaraldehyde concentration (%, v/v) | Average pore diameter (µm) | Porosity (%) |
|---|---|---|
| 0.30 | 62.453 | 61.124 |
| 0.20 | 59.381 | 67.412 |
| 0.10 | 67.144 | 64.375 |
| 0.05 | 64.455 | 63.461 |
| 0.01 | 71.299 | 63.711 |

From the SEM images of each of the groups, it can be seen that the artificial dressings in each of the groups have complete and dense porosity. After calculation by software, pore diameter is averagely distributed between 60 and 70 µm, and porosity is approximately 60% to 67%. It can be known that glutaraldehyde within this concentration range does not affect the pore diameter and porosity of the artificial dressings. The porosity of the artificial dressing can be any value in the above value range (60% to 67%) and any range.

### Embodiment 4: Water absorbency of artificial dressings

Water absorbency of the artificial dressings is tested according to standard method EN-13726-1.

It can be known from Table 3 that the 4 cm² artificial dressing can absorb liquid 25 to 35 times of its own weight.

**Table 3: Water absorbency results (I and II) of artificial dressings Result (I)**

| Dressing batch | | First batch | Water absorption multiple |
|---|---|---|---|
| First measurement | Weight after water absorption (g) | 2.027 | 30.18 |
| | Final weight (g) | 0.065 | |
| | Volume of water absorbed (mL) | 1.962 | |
| Second measurement | Weight after water absorption (g) | 1.882 | 37.41 |
| | Final weight (g) | 0.049 | |
| | Volume of water absorbed (mL) | 1.833 | |
| Third measurement | Weight after water absorption (g) | 2.138 | 37.18 |
| | Final weight (g) | 0.056 | |
| | Volume of water absorbed (mL) | 2.082 | |
| Average of three times | | 1.99 | 34.92 |

Result (II)

| Dressing batch | | Second batch | Water absorption multiple |
|---|---|---|---|
| First measurement | Weight after water absorption (g) | 1.937 | 24.83 |
| | Final weight (g) | 0.075 | |
| | Volume of water absorbed (mL) | 1.862 | |
| Second measurement | Weight after water absorption (g) | 2.021 | 30.09 |
| | Final weight (g) | 0.065 | |
| | Volume of water absorbed (mL) | 1.956 | |
| Third measurement | Weight after water absorption (g) | 2.071 | 26.61 |
| | Final weight (g) | 0.075 | |
| | Volume of water absorbed (mL) | 1.996 | |
| Average of three times | | 1.94 | 27.18 |

### Embodiment 5: Thermal disintegration testing of artificial dressings

A purpose of this thermal disintegration testing is to see whether the artificial dressings will disintegrate. Thermal disintegration tests involved soaking the artificial dressings in excess liquid volume and placing the artificial dressings in an environment with a temperature of 37°C for 24 hours under harsh conditions.

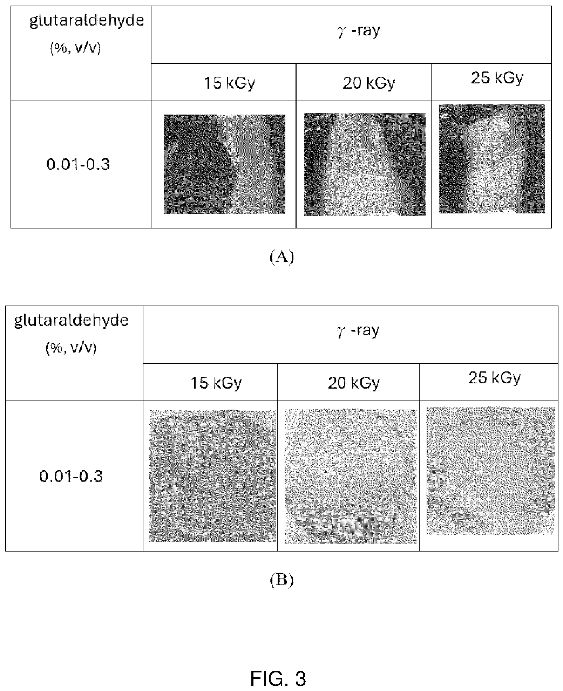

Put the artificial dressings into 4 mL of deionized water and place the artificial dressings in an incubator with a temperature of 37°C for 24 hours. After 24 hours, the artificial dressings are removed and photographed, as shown in FIG. 3 (A).

FIG. 3 (A) shows results of testing whether artificial dressings will thermally disintegrate by irradiating gamma rays (γ-ray) with different intensities.

It can be known from FIG. 3 (A) that the artificial dressings of the invention can still maintain its hydrocolloid texture and will not disintegrate after being irradiated to different radiation intensities and subjected to harsh test conditions.

### Embodiment 6: Adhesion of artificial dressings

According to the results in FIG. 3 (A), it can be known that the artificial dressings will not disintegrate. Therefore, based on the results, actual situation after the artificial dressings being applied to a hand is tested.

Adhere the 2 cm × 2 cm artificial dressing on an arm, drop 0.75 mL of saline solution on the artificial dressing, and cover it with waterproof and breathable dressing (e.g., Tegaderm by 3M) for 7 hours. After 7 hours, take another photo and observe whether it is easy to remove the artificial dressing from the arm and whether it sticks to the waterproof and breathable dressing, as shown in FIG. 3 (B).

FIG. 3 (B) shows adhesion test results of artificial dressings irradiated with γ-ray of different intensities.

It can be known from FIG. 3 (B)that the artificial dressings can be removed from the skin and Tegaderm and an appearance maintains a certain integrity and is not sticky.

### Embodiment 7: Animal experiments of the artificial dressings

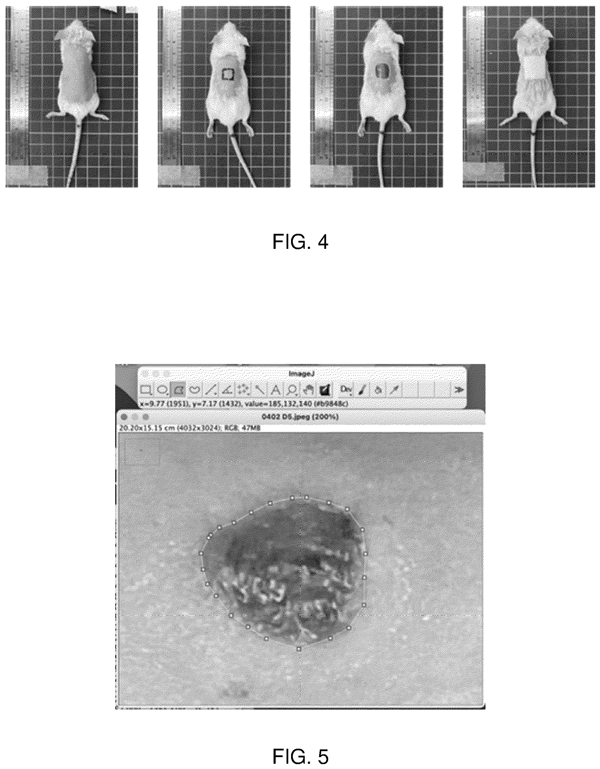

As shown in FIG. 4, wound healing by using the artificial dressings is observed through animal model of mice wounds. The day before performing trauma surgery, the mouse's back hair needs to be shaved. The above mice were purchased from the National Laboratory Animal Center in Taiwan (BALB/cByJNar1 strain). On the day of surgery, give the mouse an intraperitoneal injection (anesthetic dose: 80 mg/kg -160 mg/kg), press the hind limbs to observe pain response, and after confirming deep anesthesia, use a marker to draw a square with an area of 1*1 cm² on the back, use forceps to lift the epidermis on the back, and use surgical scissors to cut off the epidermis according to the drawn rectangle to complete the trauma surgery. One week after the surgical operation, the experimental mice are given subcutaneous injections (analgesic dose: 5 mg/kg) every day, and health status of the mice after the surgical operation is observed and recorded. After the surgical operation, the artificial dressings (1.25*1.25 cm²) are replaced and bandaged every 2 days for the experimental mice, and wound healing status is recorded at the same time. Replacement of the artificial dressings is continued for four weeks until wounds are completely closed.

### Embodiment 8: Animal experiment-wound area statistics

Import wound images into imaged software to estimate an area, use the scale bar in the image to correct and convert a pixel ratio of each of the images, and use the original skin type around the wound as a boundary standard. Circle an area of the unhealed wound in the image to calculate a size of the wound area as shown in FIG. 5.

### Embodiment 9: Animal experiments-tissue sections

It takes about 30 days from completion of the incision surgery to healing and closing of the wound in the experimental mice. After the mice are sacrificed, skin tissues of a new range from the wounds are respectively taken for embedding and staining, staining with hematoxylin-eosin (H&E). H&E pathological sections are made to observe differences between the new epidermal tissues and the original epidermal tissues. New epidermis and proliferated fibroblasts can be seen in the H&E sections, and the following three points are used as criteria for judging a degree of wound bed healing: (1) fibroblasts can synthesize and secrete collagen, so a degree of wound bed recovery can be seen from a density difference of collagen, the higher the density, the more complete the wound healing; (2) representative cells of acute inflammation are multinuclear leukocytes (neutrophil); during acute inflammation, cells will die rapidly, at this time, multinucleated leukocytes will enter the inflamed tissues and phagocytose necrotic cells or bacteria; therefore, when the sliced tissues show multinucleated leukocytes, it means that the wound is in an acute inflammation stage; and (3) representative cells of chronic inflammation are monocytes and lymphocytes; chronic inflammation is a barrier effect against pathological changes, but long-term chronic inflammation can cause connective tissue hyperplasia; monocytes and lymphocytes can be seen in the sections, indicating that the wound is in a chronic inflammation stage; if fibrous connective tissue granuloma is present, that is, scar hyperplasia, it is the result of long-term chronic inflammation.

Experiments are divided into several groups. The experimental control group uses a commercially available collagen foam dressing. The groups are shown in Table 4. In addition to comparing with the control group, in order to understand differences between using the artificial dressing dryly and wetly and the best way of using the artificial dressing, the artificial dressing in group D is moistened in advance to observe results of wound healing.

**Table 4: Grouping of experimental groups**

| | | |
|---|---|---|
| A | No dressing (NC) | Use Tegaderm alone for fixation |
| B | Commercially available dressing group (PC) | Commercially available collagen foam dressing product is selected as the control group |
| C | Artificial dressing (dry application) | The artificial dressing is directly attached to a wound without additional moisture |
| D | Artificial dressing (wet application) | The artificial dressing is moistened with 150 µL PBS |

**Table 5: Wound healing rate, standard deviation and coefficient of variation (%) from 0 to 23 days**

| Wound healing rate (Δwound healing%/day) | A | B | C | D |
|---|---|---|---|---|
| 0 day - 2 days | -9.0 | -17.5 | -5.0 | 1.0 |
| 2 days - 4 days | -6.0 | -5.0 | -5.0 | -6.0 |
| 4 days - 6 days | -9.0 | -2.5 | -5.0 | -6.0 |
| 6 days - 8 days | -7.0 | -2.5 | -7.5 | -1.5 |
| 8 days - 10 days | -1.5 | -10.0 | -1.5 | -5.0 |
| 10 days-12 days | -1.5 | -3.5 | -6.0 | -9.0 |
| 12 days-14 days | -2.0 | -1.0 | -5.0 | -4.5 |
| 14 days-16 days | -1.5 | -1.0 | -1.0 | -4.0 |
| 16 days-18 days | -2.5 | -1.0 | -3.0 | -1.0 |
| 18 days-20 days | -1.0 | -0.5 | -3.5 | -1.5 |
| 20 days-23 days | -2.7 | -0.3 | -1.7 | -1.7 |
| Average wound healing rate | -3.97 | -4.07 | -4.02 | -3.56 |
| Standard deviation | 3.0 | 5.0 | 2.0 | 2.8 |
| Coefficient of variation (%) | 75.39 | 123.19 | 48.65 | 77.52 |

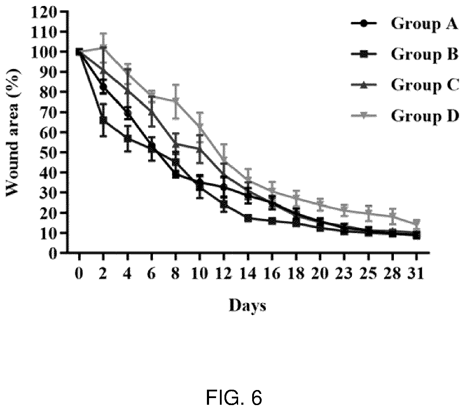

FIG. 6 shows that wound areas in group A to group D are close to healing on the 23rd day. Table 5 shows the wound healing rate, standard deviation and coefficient of variation calculated based on FIG. 6. The wound healing rate is a percentage of wound area closing in each period of time; the standard deviation and coefficient of variation indicate a degree of change in the wound healing rate. The smaller a numerical value of the standard deviation and coefficient of variation, the smaller a degree of change in the wound healing rate, indicating that the wound healing rate in the group is relatively stable, and a stable wound healing rate will promote wound healing. The coefficients of variation of the no dressing group and the artificial dressing wet application group (groups A and D) are similar, indicating that a stability of the wound healing rate in the artificial dressing wet application group is similar to that of the no dressing group. The commercially available collagen dressing group (Group B) has a fast wound healing rate in an initial stage, but the overall coefficient of variation is the highest among the four groups, indicating that a wound healing process is relatively unstable. The artificial dressing dry application group (Group C) has the smallest coefficient of variation among the four groups, highlighting that the wound is the most stable in a healing stage and has the best prognosis.

### Embodiment 10: Animal experiments-tissue section results analysis

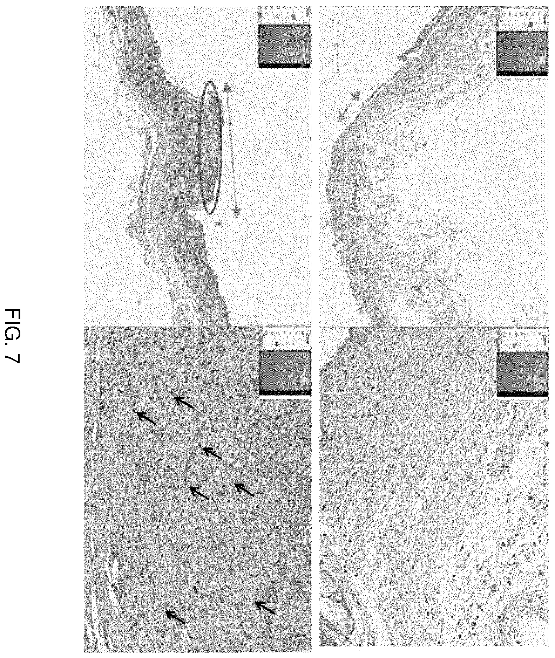

Tissue section results of the no dressing group (Group A) are shown in FIG. 7. There is fibrosis in the wound bed, hyperplasia of granuloma on the surface, and multinuclear leukocytes (neutrophil) are still present deeply in the tissues, indicating that it is still in an acute inflammatory stage.

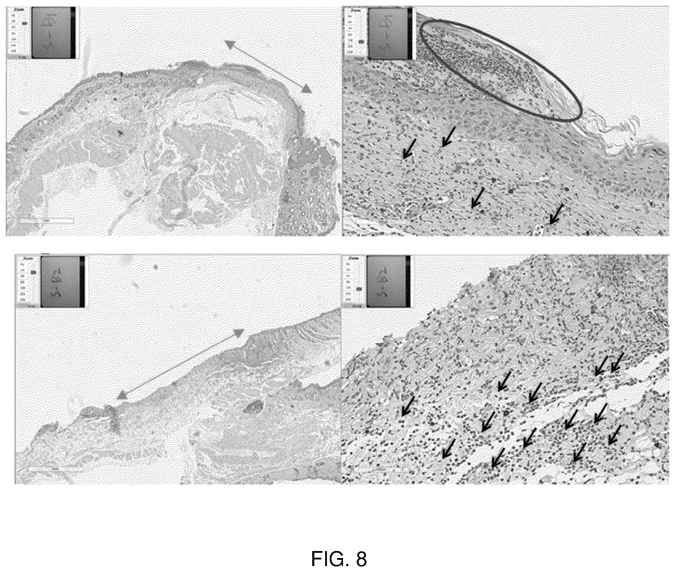

Tissue section results of the commercially available dressing group (Group B) are shown in FIG. 8. There is fibrosis in the wound bed also, but a structure is relatively loose, hyperplasia of granuloma on the surface, and multinuclear leukocytes (neutrophil) are still present deeply in the tissues, indicating that it is still in an acute inflammatory stage.

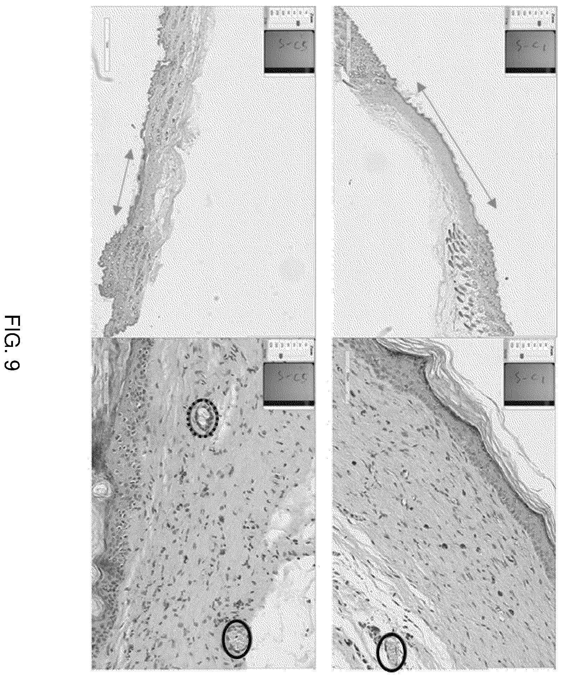

Tissue section results of the artificial dressing dry application group (Group C) are shown in FIG. 9. The wound bed has significant tissue arrangements of light fibroblasts and collagen. The wound is fibrotic and has a dense structure. No inflammatory reactions such as leukocytes are observed. The skin surface is intact, and some new functional tissues and blood vessels can even be observed. It is estimated that the wound has reached late proliferation and remodeling stages.

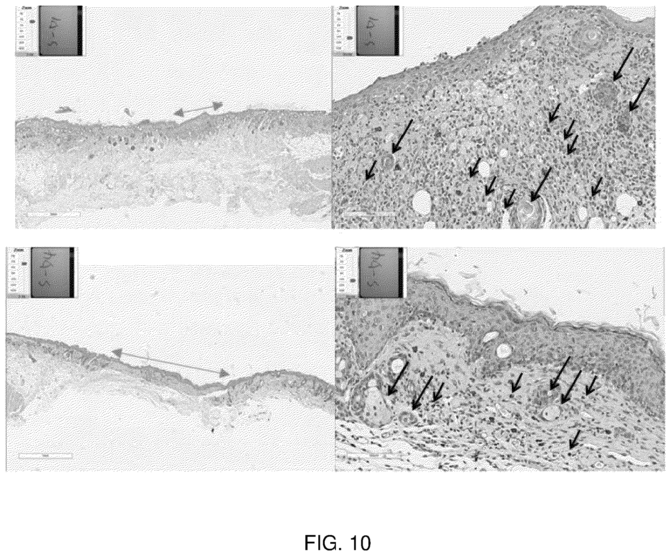

Tissue section results of the artificial dressing wet application group (Group D) are shown in FIG. 10. There is slight fibrosis and granuloma in the wound bed. Functional tissue hyperplasia of the skin is also observed, and distribution of monocytes is observed below the wound bed, it is estimated that the wound is in a late stage of inflammation.

Based on results of wound healing and tissue sections, internal tissues of the no dressing group (Group A) and the commercially available dressing group (Group B) are still in an acute inflammatory stage, a fibrotic tissue structure is relatively loose, and there is growth of granuloma on a surface, making scars relatively easy to appear. Compared with the artificial dressing dry application group (Group C), there is no inflammatory reaction in internal tissues, and some new functional tissues and blood vessels can even be observed. Structures of wound fibrosis tissues are dense, the skin surface is intact, and no scars are produced.

If wound healing conditions of the artificial dressing dry application group and the artificial dressing wet application group (groups C and D) are compared, a wound healing rate of the artificial dressing dry application group (group C) is faster through the dressing itself absorbing tissue fluid; while the artificial dressing wet application group (Group D) has a larger number of new structural tissues and blood vessels. A reason is speculated that chronic inflammatory reaction will promote new functional tissues and blood vessels of the skin.

Results of tissue sections are consistent with results in Table 5. In Table 5, the artificial dressing dry application group (Group C) has the smallest coefficient of variation and the most stable wound healing rate. Compared with section results in FIG. 9, the artificial dressing dry application group (Group C) has the best conditions after healing. An initial wound healing rate of the commercially available dressing group (Group B) is fast, but an overall coefficient of variation is the highest. Compared with the section results in FIG. 8, although granuloma has been formed on a wound surface, deeply in the tissues are still in an acute inflammation stage, indicating that the wound is not completely healed. In order to promote wound healing, in addition to a stable healing rate, densely structured fibrotic tissues are also required. Therefore, the artificial dressing of the invention conforms to the above-mentioned viewpoints and is capable of promoting wound healing.

### Embodiment 11: usage of artificial dressing

According to the experimental results, the best way to use the artificial dressing of the invention is: in an early stage of wound healing, apply the artificial dressing dryly and directly to promote the growth of fibroblasts and collagen; in a later stage of wound healing, when tissue fluid is relatively reduced, add a small amount of water to wet the artificial dressing to promote functional tissues and angiogenesis in the epidermis, providing the wound with the most complete care. The invention provides an artificial dressing suitable for promoting wound healing, which comprises a composition of a gelatin, a polysorbate 20 (tween 20) and a glutaraldehyde. The artificial dressing is suitable for moisture therapy. The artificial dressing of the invention is capable of: absorbing tissue fluid of the wound; inhibiting proliferation of granuloma of the wound; reducing inflammation of the wound; promoting angiogenesis in the wound tissues; and reducing adhesiveness of the wound to promote wound healing. The wound includes but is not limited to bruises, contusions, cuts, general acute wounds, first/second-level burns, surgical wounds, bedsores and ulcers.

Although the invention has been described and illustrated in sufficient detail to enable those skilled in the art to make and use the artificial dressing, various substitutions, modifications and improvements are apparent without departing from the spirit and scope of the invention.

Accordingly, an artificial dressing, use of artificial dressing for promoting wound healing and method of manufacturing artificial dressing provide the following advantages:
(1). The artificial gelatin dressing has obvious pores and is capable of absorbing a liquid weight about 25-35 times of its body weight.
(2). The artificial dressing of the invention is capable of accelerating wound healing when applied dryly; and capable of proliferating functional tissues of the wound when applied wetly.
(3). A main component used in an artificial dressing of the invention is gelatin, which has the advantages of biocompatibility, low cost, and an ability to absorb water and form gel. The cost of the artificial dressing of the invention is far lower than the traditional moisture therapy and dressings.
(4). The artificial dressing of the invention is capable of: absorbing tissue fluid of the wound; inhibiting proliferation of granuloma of the wound; reducing inflammation of the wound; promoting angiogenesis in the wound tissues; and reducing adhesiveness of the wound to promote wound healing.

It is readily apparent to those skilled in the art that the invention is well adapted to carry out the above objects and obtain the above objects and advantages, as well as those inherent therein. Processes and methods for producing the artificial dressing described above represent preferred embodiments, are illustrative, and do not limit the scope of the invention. Any person having ordinary skill in the art can come up with modifications and other uses. The modifications are included in the spirit of the invention and are defined by the scope of the claims.

## Claims

1. An artificial dressing, suitable for promoting wound healing, comprising a gelatin, a polysorbate 20 (tween 20) and a glutaraldehyde.

2. The artificial dressing as claimed in claim 1, wherein the concentration of gelatin is 5% (g/mL, w/v) to 10% (g/mL, w/v), the concentration of polysorbate 20 is 0.005% (mL/mL, v/v) ~ 0.1% (mUmL, v/v), the concentration of glutaraldehyde is 0.01% (mUmL, v/v) ~ 0.3% (mUmL, v/v) to form the artificial dressing.

3. The artificial dressing as claimed in claim 1, wherein a thickness of the artificial dressing is 1-10 mm.

4. The artificial dressing as claimed in claim 1, wherein a pore diameter of the artificial dressing is 60-70 µm.

5. The artificial dressing as claimed in claim 1, wherein a porosity of the artificial dressing is 60%-67%.

6. The artificial dressing as claimed in claim 1, wherein the wound includes, but is not limited to, bruises, contusions, cuts, general acute wounds, first/second-level burns, surgical wounds, bedsores and ulcers.

7. The artificial dressing as claimed in claim 1, wherein the artificial dressing is in a sheet shape.

8. The artificial dressing as claimed in claim 1, wherein the artificial dressing becoming gelatinous when the artificial dressing being humidified or absorbing tissue fluid.

9. The artificial dressing as claimed in claim 1, wherein the artificial dressing has a tolerance to high-energy gamma radiation exposure doses of 15 kGy-25 kGy without affecting texture and function.

10. A method for manufacturing the artificial dressing as claimed in claim 1, comprising:
(1) performing a foaming step by mixing a gelatin, a glutaraldehyde and a polysorbate 20 to form a mixture and stirring to foam;
(2) performing a molding step by pouring the mixture onto a mold and spreading it flat after completing the foaming step; and
(3) performing a freezing step by placing the mold into a pre-cooled (pre-cooling temperature -20°C~-60°C) plate-type freeze dryer for freezing and freeze-drying to form an artificial dressing.

11. The method as claimed in claim 10, wherein a concentration of the gelatin is 5% (w/v)-10% (w/v).

12. The method as claimed in claim 10, wherein a concentration of the polysorbate 20 is 0.005% (v/v)~0.1% (v/v).

13. The method as claimed in claim 10, wherein a concentration of the glutaraldehyde is 0.01% (v/v)-0.3% (v/v).

14. A method of using a composition in promotion of wound healing, comprising applying the artificial dressing as claimed in claim 1, which is acted as the composition, to a patient's wound.

15. The method as claimed in claim 14, wherein the composition is used for moisture therapy.

16. A use of a composition in preparing an artificial dressing for promoting wound healing, comprises providing a composition, which comprises a gelatin, a polysorbate 20 and a glutaraldehyde; and performing a foaming step, a molding step, and a freeing step on the composition.

17. The use as claimed in claim 16, wherein the concentration of gelatin is 5% (g/mL, w/v) to 10% (g/mL, w/v), the concentration of polysorbate 20 is 0.005% (mUmL, v/v) ~ 0.1% (mL/mL, v/v), the concentration of glutaraldehyde is 0.01% (mL/mL, v/v) ~ 0.3% (mUmL, v/v) to form the artificial dressing.

18. The use as claimed in claim 16, wherein the composition is capable of: (1) absorbing tissue fluid of the wound; (2) inhibiting proliferation of granuloma of the wound; (3) reducing inflammation of the wound; (4) promoting angiogenesis in the wound tissues; and (5) reducing adhesiveness of the wound to promote wound healing.

19. The use as claimed in claim 16, wherein the wound includes bruises, contusions, cuts, general acute wounds, first/second-level burns, surgical wounds, bedsores and ulcers.
